# EUROPEAN PATENT SPECIFICATION

(11) **EP 3 888 575 B1**
(45) Date of publication and mention of the grant of the patent: **25.12.2024**
(21) Application number: 19888934.7
(22) Date of filing: 18.11.2019
(51) Int. Cl.: A61B 17/435

(54) **EMBRYO TRANSPLANTING TOOL AND EMBRYO TRANSPLANTING APPARATUS**
EMBRYOTRANSPLANTATIONSWERKZEUG UND EMBRYOTRANSPLANTATIONSVORRICHTUNG
OUTIL DE TRANSPLANTATION D'EMBRYONS ET APPAREIL DE TRANSPLANTATION D'EMBRYONS

(30) Priority: 29.11.2018 JP 2018223434
(43) Date of publication of application: 06.10.2021
(73) Proprietor: Kitazato Corporation, Fuji-shi, Shizuoka 416-0932 (JP)
(72) Inventor: INOUE, Futoshi, Fuji-shi, Shizuoka 416-0932 (JP)
(74) Representative: Casalonga
(86) International application number: PCT/JP2019/045145
(87) International publication number: WO 2020/110813

(56) References cited:
- EP-A1- 3 345 559
- WO-A1-2017/038557
- WO-A1-2019/136146
- JP-A- 2004 298 632
- JP-A- H02 268 745
- US-A1- 2003 040 756
- US-A1- 2004 193 055
- ANONYMOUS: "Glass microsphere - Wikipedia", 13 November 2018 (2018-11-13), XP055943499, Retrieved from the Internet <URL:https://en.wikipedia.org/w/index.php?title=Glass_microsphere&oldid=868567763> [retrieved on 20220718]

## Description

### TECHNICAL FIELD

The present invention relates to an embryo transfer tool and an embryo transfer device. More specifically, the present invention relates to an embryo transfer tool and an embryo transfer device to be used in transferring an embryo (fertilized ovum) fertilized in vitro to the uterus of a living body, namely, to the uterus of an animal.

### BACKGROUND ART

In transferring an embryo into the uterus, it is general to collect ova and sperms, put the ova in a culture vessel, add a sperm suspension to the ova, namely, fertilize the ova with sperms, and transfer a fertilized ovum or an embryo obtained by dividing the fertilized ovum into two parts, four parts or eight parts to the uterus of an animal, for example, a human uterus.

Thereafter a comparatively hard sheath is inserted into the uterus from the vaginal opening and passed through the cervical canal. Thereafter the embryo is directly inserted into the sheath or a tube which has sucked the embryo thereto is inserted thereinto. Thereafter the tube is pressed into the sheath. After the front end portion of the tube reaches the cervical opening, a syringe is pressed to transfer the embryo into the uterus. Thereafter the tube and a mantle tube are removed from the sheath. In this manner, the transfer of the embryo finishes.

The present inventors proposed an embryo transfer device as disclosed in Japanese Patent Application Laid-Open Publication No. 2004-129789 (patent document 1). The embryo transfer device 1 of the patent document 1 has the flexible sheath 2 having the path penetrating therethrough from its front end to its rear end and the spherical bulged part 22 provided on the outer surface of the front end thereof, the flexible stylet 3 which is removably inserted into the flexible sheath and whose front end projects a little from the front end surface of the sheath, and the transfer tube body 4 having he flexible front end part 41a which can be inserted into the flexible sheath 2 from which the stylet has been removed and can be projected in a predetermined length from the front end of the flexible sheath 2.

The transfer tube body 4 to be used for the embryo transfer device is desired to have a high ultrasound imaging property at its front end portion. The transfer tube bodies having a high ultrasound imaging property are proposed, as disclosed in Japanese Patent Application Laid-Open Publication No. 2003-190275 (patent document 2) and Japanese Patent Application Laid-Open Publication No. 2004-298632 (patent document 3). The present inventors proposed WO2017/038557 (patent document 4).

In the surgical medical device (1, 1') made of a plastic material disclosed in the patent document 2, the plastic material contains gas bubbles (12, 12') in the major part of the thickness thereof at least one selected portion of the device to allow the device to have visibility in an ultrasound imaging operation.

The embryo replacing catheter having the flexible shaft 1 formed by press molding transparent polyurethane is also disclosed in the patent document 2. The shaft 1 has the hole 10 extended longitudinally. The gas bubbles 12 whose diameters are in the range of 5µ to 10µ are introduced into the wall of the shaft in the thickness direction thereof by adding a gas to the transparent polyurethane while it is being press molded. In the disclosure, the number of the bubbles 12 is so selected as to increase the visibility of the catheter while the ultrasound imaging operation is being performed and view a substance flowing along the catheter.

The catheter for transporting an embryo or another medical device disclosed in the patent document 3 has the shaft 1 having two layers 12 and 13 formed by press molding. The outer layer 13 is comparatively thick and contains the bubbles 22 whose number is large enough to improve the visibility of the catheter in ultrasound observation. The density of the bubbles is so set that the substance inside the catheter can be viewed with the naked eye. The inner layer 12 is comparatively thin and does not contain bubbles in order to allow the catheter to have the smooth hole 10.

The embryo transfer device (1) disclosed in the patent document 4 has a flexible tube (11) and a hub (12). The flexible tube (11) has a first bubble-containing surface layer (13a) extended in a predetermined width and in a predetermined length from a distal end of the flexible tube toward a proximal end thereof, a second bubble-containing surface layer (13b) opposed to the first bubble-containing surface layer (13a), and first and second colorless and transparent parts (15a) and (15b) positioned between the first and second bubble-containing surface layers (13a) and (13b). Each of the first and second bubble-containing surface layers has a lot of bubbles (14) set long in an axial direction of the flexible tube. The thickness of each of the first and second bubble-containing surface layers is set to 1/5 to 1/3 of the thickness of the flexible tube. The width of each of the first and second bubble-containing surface layers is set to 5/100 to 20/100 of an outer circumferential length of the flexible tube.

### PRIOR ART DOCUMENTS

### PATENT DOCUMENTS

Patent document 1: Japanese Patent Application Laid-Open Publication No. 2004-129789
Patent document 2: Japanese Patent Application Laid-Open Publication No. 2003-190275 (USP8092390)
Patent document 3: Japanese Patent Application Laid-Open Publication No. 2004-298632 (USP10045756)
Patent document 4: WO2017/038557(EP3345559) Patent document 5: WO2019/136146 is prior art pursuant to Art. 54(3) EPC and discloses a catheter having echogenic properties by introducing stripes comprising glass beads.

### SUMMARY OF THE INVENTION

### PROBLEMS TO BE SOLVED BY THE INVENTION

Each of the transfer tube bodies of the patent documents 2, 3, and 4 has a sufficiently high ultrasound imaging property. However, it is difficult to make bubbles be contained in an inner wall of a tube in a favorable manner, and irregularities are likely to be formed in the outer surface of the tube due to bursting of the bubbles, and there are also cases where the distribution of the bubbles is not uniform and the nonuniformity appears as a contrast in ultrasound imaging.

It is an object of the present invention to provide an embryo transfer tool in which the inner surface and the outer surface of a tube both have good smoothness, and which has a uniform and sufficiently high ultrasound imaging property and has sufficiently high optical transparency and visibility when microscopically observing the inside thereof by using a light source, and to provide an embryo transfer device using the embryo transfer tool.

### MEANS FOR SOLVING THE PROBLEMS

The above-described object can be achieved by an embryo transfer tool according to claim 1. The dependent claims relate to advantageous embodiments.

### BRIEF DESCRIPTION OF THE DRAWINGS

Fig. 1 is a front view of one embodiment of an embryo transfer tool of the present invention.
Fig. 2 is a front view of an embryo transfer device using the embryo transfer tool shown in Fig. 1.
Fig. 3 is a front view of a sheath used for the embryo transfer device shown in Fig. 2.
Fig. 4 is an enlarged view of a distal end portion of the embryo transfer device shown in Fig. 2.
Fig. 5 is an enlarged view of a distal end portion of the embryo transfer tool shown in Fig. 1.
Fig. 6 is a sectional view taken along a line A-A of Fig. 5.
Fig. 7 is an enlarged sectional view of a proximal end portion of the embryo transfer device shown in Fig. 2.
Fig. 8 is a front view of another embodiment of an embryo transfer tool of the present invention.
Fig. 9 is an enlarged view of a distal end portion of the embryo transfer tool shown in Fig. 8.
Fig. 10 is an enlarged side view of a distal end portion of the embryo transfer tool shown in Fig. 8.

### MODE FOR CARRYING OUT THE INVENTION

The embryo transfer tool of the present invention and the embryo transfer device of the present invention using the embryo transfer tool are described below by using embodiments shown in the drawings.

An embryo transfer tool 1 of the present invention includes a flexible tube 11 that is made of a colorless and transparent synthetic resin, and a hub 12 that is provided at a proximal end portion of the flexible tube 11. The flexible tube 11 includes a first microparticle-containing portion 13a that has a predetermined width and extends over a predetermined length from a distal end portion of the flexible tube toward a proximal end thereof, a second microparticle-containing portion 13b that has a predetermined width, extends from the distal end portion toward the proximal end, and opposed to the first microparticle-containing portion 13a, a first colorless and transparent portion 15a that is positioned between the first microparticle-containing portion 13a and the second microparticle-containing portion 13b, extends over a predetermined length from a distal end of the flexible tube 11 toward the proximal end thereof, and allows the inside of a lumen to be visually recognized, and a second colorless and transparent portion 15b that is provided so as to opposed to the first colorless and transparent portion 15a. The first and second microparticle-containing portions 13a and 13b are positioned within an inner wall of the flexible tube 11, are not exposed in the outer surface and the inner surface of the flexible tube 11, and are constituted by a synthetic resin and a large number of microparticles 14 that are formed of a material different from the synthetic resin. The microparticles 14 are dispersed in the synthetic resin, and the first and second microparticle-containing portions 13a and 13b include a large number of boundary surfaces that are formed between the synthetic resin and the microparticles.

The embryo transfer tool 1 of this embodiment has the flexible tube 11 having the lumen penetrating therethrough from its distal end to its proximal end and the hub 12 fixed to the proximal end portion of the flexible tube 11. The hub 12 may be formed integrally with the flexible tube.

The flexible tube 11 serves as a means for transferring an embryo and has the lumen 16 penetrating therethrough from its distal end to its proximal end. The length of the flexible tube is set to 70 to 800mm and favorably 200 to 600mm. The outer diameter of the flexible tube is set to 0.5 to 3mm and favorably 1 to 2mm. The inner diameter of the flexible tube is set to 0.3 to 0.7mm and favorably 0.4 to 0.6mm.

As materials for forming the flexible tube 11, those having colorless, transparency and flexibility to a certain extent are preferable. It is possible to use synthetic rubber such as urethane rubber, silicone rubber, butadiene rubber, soft vinyl chloride, polyolefin (polyethylene, polypropylene, ethylene-propylene copolymer, ethylene-vinyl acetate copolymer, mixture of polypropylene and polyethylene or mixture of polypropylene and polybutene), polyester (polyethylene terephthalate, polybutylene terephthalate), polyamide, elastomers such as polyolefin-based elastomer, polyamide-based elastomer, styrene-based elastomer (for example, styrene-butadiene-styrene copolymer, styrene-isoprene-styrene copolymer, styrene-ethylene-butylene-styrene copolymer), polyurethane, and preferably, thermoplastic polyurethane (thermoplastic polyether polyurethane and thermoplastic polyester polyurethane are preferable. Segmented thermoplastic polyether polyurethane having a soft segment portion and a hard segment portion are especially preferable. More specifically, as a main component of the soft segment, polytetramethylene ether glycol, polyethylene glycol, and polypropylene glycol are preferable. As a main component of the hard segment, 1,4-butanediol is preferable). Polyamides or polyamide elastomers are preferred.

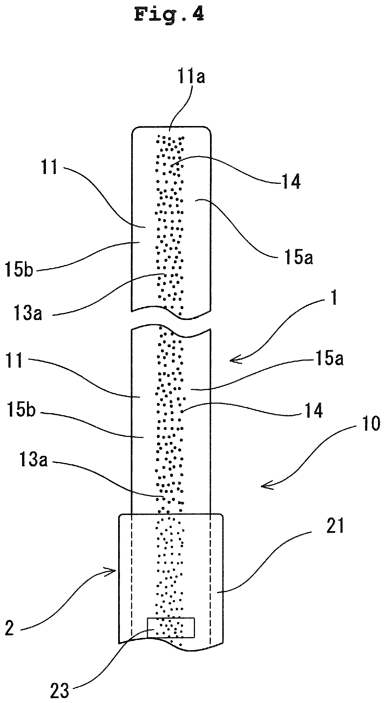

As shown in Figs. 4 through 6, the flexible tube 11 has the first microparticle-containing portion 13a extended in the predetermined width and in the predetermined length from the distal end of the flexible tube toward the proximal end thereof and the second microparticle-containing portion 13b extended in the predetermined width from the distal end of the flexible tube toward the proximal end thereof and opposed to the first microparticle-containing portion 13a through the intermediary of the center of the flexible tube 11. In the embryo transfer tool 1 of this embodiment, the first and second microparticle-containing portions 13a and 13b are extended from the distal end of the flexible tube 11 to the proximal end thereof. As shown in Fig. 4, it is necessary for the flexible tube to have the first and second microparticle-containing portions 13a and 13b at a part projected from a distal end of the flexible tube 21 of the sheath 2. The first and second microparticle-containing portions may not necessarily be formed at a part which is located proximally from the part projected from flexible tube.

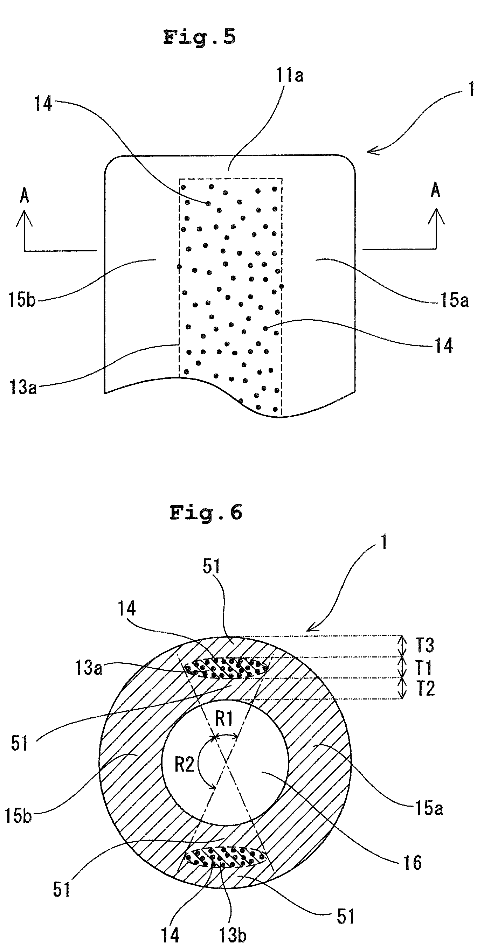

As shown in Fig. 6, the first and second microparticle-containing portions 13a and 13b are positioned within the inner wall of the flexible tube 11 and are not exposed in the outer surface and the inner surface of the flexible tube 11. Also, the first and second microparticle-containing portions 13a and 13b are constituted by the synthetic resin and the large number of microparticles 14 that are formed of the material different from the synthetic resin. The microparticles 14 are substantially uniformly dispersed in the synthetic resin. The first and second microparticle-containing portions 13a and 13b include the large number of boundary surfaces formed between the synthetic resin and the microparticles 14, specifically, formed by surfaces of the microparticles 14. Ultrasonic waves reflect off boundary surfaces that are formed by different substances, and therefore, the first and second microparticle-containing portions 13a and 13b containing the microparticles can be imaged by ultrasonic echoes, and positions of the microparticle-containing portions in a living body can be easily checked.

As the synthetic resin used for the first and second microparticle-containing portions 13a and 13b, those having transparency and flexibility to a certain extent are preferable. It is possible to use synthetic rubber such as urethane rubber, silicone rubber, butadiene rubber, soft vinyl chloride, polyolefin (polyethylene, polypropylene, ethylene-propylene copolymer, ethylene-vinyl acetate copolymer, mixture of polypropylene and polyethylene or mixture of polypropylene and polybutene), polyester (polyethylene terephthalate, polybutylene terephthalate), polyamide, elastomers such as polyolefin-based elastomer, polyamide-based elastomer, styrene-based elastomer (for example, styrene-butadiene-styrene copolymer, styrene-isoprene-styrene copolymer, styrene-ethylene-butylene-styrene copolymer), polyurethane or polyurethane elastomer, for example, thermoplastic polyurethane (thermoplastic polyether polyurethane and thermoplastic polyester polyurethane are preferable. Segmented thermoplastic polyether polyurethane having a soft segment portion and a hard segment portion are especially preferable. More specifically, as a main component of the soft segment, polytetramethylene ether glycol, polyethylene glycol, and polypropylene glycol are preferable. As a main component of the hard segment, 1,4-butanediol is preferable). Polyurethane, polyurethane elastomer, polyurethane and polyamide elastomers are preferred.

The synthetic resin used in the first and second microparticle-containing portions 13a and 13b may be transparent and colored. As the coloring, blue, green, gray and the like are preferable.

The synthetic resin forming the first and second microparticle-containing portions 13a and 13b is a synthetic resin that has higher flexibility than the synthetic resin forming the flexible tube 11. Due to containing microparticles, the material forming the first and second microparticle-containing portions 13a and 13b is highly likely to have a lower flexibility than in the case of not containing microparticles. Therefore, if the above configuration is employed, it is possible to reduce a difference in physical properties between the material forming the first and second microparticle-containing portions 13a and 13b and the material forming the flexible tube. In particular, the material forming the first and second microparticle-containing portions, which is constituted by the microparticles and the synthetic resin, has flexibility that is substantially equivalent to the flexibility of the synthetic resin (material) forming the flexible tube. The flexibility can be adjusted by adjusting the synthetic resin forming the first and second microparticle-containing portions 13a and 13b and the amount of microparticles added to the synthetic resin. This configuration further reduces the difference in physical properties between the material forming the first and second microparticle-containing portions 13a and 13b and the material forming the flexible tube.

In the case of the present invention where different materials are selected as a microparticle-free portion forming resin (first resin) and a microparticle-containing portion forming resin (second resin), it is preferable to select materials that have good compatibility with each other as the first resin and the second resin from the standpoint of coextrusion moldability. Having good compatibility means that the thermodynamic mutual solubility of the materials is good, in other words, the materials do not separate from each other after being cured. A resin that has higher flexibility (is softer) than the first resin is selected as the second resin.

It is desirable to select resins that are of the same type and have different physical properties, as the first resin and the second resin. Examples of types of resin include polyurethane-based resin, polyolefin-based resin, polyamide-based resin, and polyester-based resin.

More specifically, it is conceivable to: select a soft polyurethane as the first resin and select a polyurethane that is softer than the first resin, as the second resin; select a polyamide elastomer as the first resin and select a polyamide elastomer that is softer than the first resin, as the second resin; select a polyolefin-based elastomer (e.g., a polyethylene elastomer) as the first resin and select a polyolefin-based elastomer (e.g., a polyethylene elastomer) that is softer than the first resin, as the second resin; or select a polyester-based elastomer (e.g., a polyester elastomer) as the first resin and select a polyester-based elastomer (e.g., a polyester elastomer) that is softer than the first resin, as the second resin. Note that it is thought that, at boundary portions between the first resin and the second resin, the first resin and the second resin are in a mixed state of being microscopically mixed as a result of a combination of resins that have high compatibility with each other being selected as the first resin and the second resin. It is thought that, accordingly, there is no interface in the strict sense between the first resin and the second resin, and boundary portions are formed by a mixture of the first resin and the second resin.

The microparticles contained in the first and second microparticle-containing portions 13a and 13b are optically transparent glass beads. The microparticles are solid and have a diameter of 0.5 to 200 µm. In particular, it is preferable that the diameter is 1 to 150 µm, or more specifically 10 to 120 µm. The microparticles may be transparent and colored. In the case where the microparticles are colored, preferable examples of the color include blue, green, and gray.

Preferable examples of optically transparent resin beads include silicone resin particles, acrylic resin particles, nylon resin particles, urethane resin particles, styrene resin particles, polyethylene resin particles, and polyester resin particles. It is preferable to select, as optically transparent resin microparticles, microparticles that are formed of a material of a different type from the microparticle-containing portion forming resin.

The microparticles contained in the first and second microparticle-containing portions 13a and 13b may be hollow microparticles, in particular, hollow microspheres. It is preferable that the hollow microparticles are hollow glass beads (hollow glass microspheres) or hollow resin beads (hollow resin microspheres). It is also preferable that the hollow microparticles are optically transparent microparticles. In particular, optically transparent hollow glass beads (optically transparent hollow glass microspheres) and optically transparent hollow synthetic resin beads (optically transparent hollow synthetic resin microspheres) are preferable as the hollow microparticles. Microparticles that have internal spaces and have a diameter of 0.5 to 200 µm are preferable as the hollow microparticles. In particular, it is preferable that the diameter is 1 to 150 µm, or more specifically 10 to 120 µm. The hollow microparticles may be transparent and colored. In the case where the hollow microparticles are colored, preferable examples of the color include blue, green, and gray.

Hollow glass microparticles (hollow glass microspheres) that have an average diameter smaller than about 500 µm are commonly known as "glass microbubbles", "glass bubbles", "hollow glass beads", or "glass balloons".

Hollow glass microparticles (hollow glass microspheres) of various sizes can be used in the embryo transfer tool of the present invention. The term "size" used here is considered to be equal to the diameter and the height of the hollow microspheres. It is preferable that the volume median diameter of the hollow glass microspheres is 10 to 80 µm, or in particular within the range of 30 to 70 µm. The volume median diameter is also called the D50 diameter, and indicates that 50 volume% of the hollow microspheres in a particle size distribution are smaller than that diameter. The volume median diameter is determined through laser diffraction by dispersing the hollow glass microspheres in degassed deionized water. For example, "MASTERSIZER 2000" (product name) manufactured by Malvern Instruments, Malvern, UK can be used as a laser diffraction particle size analyzer. Also, it is preferable that the average particle size of the hollow glass microspheres is 10 to 80 µm, or in particular within the range of 30 to 70 µm.

The hollow microspheres (hollow glass beads) that are used need to have sufficiently high strength in order to remain when the flexible tube is extrusion molded. It is preferable that the effective hydrostatic pressure (90% yield pressure resistance) under which 10 volume% of the hollow microspheres collapse is at least 3 megapascals (MPa), and preferably at least 10 megapascals (MPa). Note that each of the above numerical values of pressure resistance means the numerical value MPa ±5%. The 90% yield pressure resistance may be at least 100 MPa. The collapse strength of the hollow microspheres (hollow glass beads) is preferably measured for a dispersion of the hollow glass microspheres in glycerol using ASTM D3102-72 "Hydrostatic Collapse Strength of Hollow Glass Microspheres"; with the exception that the sample size (in grams) is equal to 10 times the density of the glass bubbles, for example.

Examples of hollow glass beads (microspheres) that can be used in the present invention include "3M GLASS BUBBLES" (product name) manufactured by 3M Company, St. Paul, MN (e.g., grades S60, S60HS, iM30K, iM16K, S38HS, S38XHS, K42HS, K46, and H50/10000), "SPHERICEL" (registered trademark, material: borosilicate glass) manufactured by Potters-Ballotini Co., Ltd., product No. 25P45 (average particle size: 45µm, pressure resistance: 5 Mpa) and product No. 60P18 (average particle size: 18 µm, particle size range: 5 to 35 µm, pressure resistance: 55 Mpa), "Q-CEL" (registered trademark, material: sodium borosilicate glass) manufactured by Potters-Ballotini Co., Ltd., product No. 5020FPS (average particle size: 40 µm, particle size range: 5 to 90 µm, pressure resistance: 3.4 Mpa), product No. 7040S (average particle size: 45 µm, particle size range: 5 to 90 µm, pressure resistance: 13.8 Mpa), and product No. 5020 (average particle size: 60 µm, particle size range: 5 to 115 µm, pressure resistance: 3.4 Mpa), "SIL-CELL" (product name) manufactured by Silbrico Corp., Hodgkins, IL (e.g., grades SIL35/34, SIL-32, SIL-42, and SIL-43), and "Y8000" (product name) manufactured by Sinosteel Maanshan Inst. of Mining Research Co., Maanshan, China.

Examples of resin materials used for the hollow resin beads (microspheres) include acrylic resin, styrene resin, acrylic-styrene copolymer resin, acrylic-acrylonitrile copolymer resin, acrylic-styrene-acrylonitrile copolymer resin, acrylonitrile-methacrylonitrile copolymer resin, acrylic-acrylonitrile-methacrylonitrile copolymer resin, vinylidene chloride-acrylonitrile copolymer resin, polymethyl methacrylate, and crosslinked polymethyl methacrylate. Any one or two or more of these resin materials can be used.

Examples of the shape of hollow microparticles include a spherical shape, an elliptical spherical shape, and a flattened spherical shape, and the spherical shape is preferable.

The ratio of the amount of microparticles contained in the first and second microparticle-containing portions 13a and 13b is preferably 0.1 to 30%, and particularly preferably 0.5 to 10%. The ratio of the amount of microparticles is more preferably 1 to 5%.

The ratio of the volume of microparticles in the first and second microparticle-containing portions 13a and 13b is preferably 1 to 30%, and particularly preferably 5 to 20%. The ratio of the volume of microparticles is more preferably 7 to 15%.

As shown in Figs. 4 and 5, the embryo transfer tool 1 of the present embodiment includes, at its distal end portion, an annular transparent distal end portion 11a that does not include the first and second microparticle-containing portions. Therefore, the first and second microparticle-containing portions 13a and 13b are not exposed in the distal end surface of the embryo transfer tool 1 (flexible tube 11) as well.

The thickness of each of the first and second microparticle-containing portions 13a and 13b is preferably 1/5 to 1/3 of the thickness of the flexible tube 11. As a result of the two microparticle-containing portions being provided so as to opposed to each other, a sufficiently high ultrasound imaging property is achieved. The width of each of the first and second microparticle-containing portions 13a and 13b is preferably at least 30/100 of the outer circumferential length of the flexible tube 11.

Furthermore, as shown in Fig. 6, a thickness T1 of each of the first and second microparticle-containing portions 13a and 13b is preferably 1/5 to 1/3 of the thickness of the flexible tube 11. In particular, the thickness T1 is preferably 1/5 to 1/4 of the thickness of the flexible tube 11. Portions that are on the upper side and the lower side of the first and second microparticle-containing portions 13a and 13b are microparticle-free portions. As a result of the thicknesses of the first and second microparticle-containing portions 13a and 13b being set as described above, physical properties of the flexible tube 11 are not largely changed due to the microparticle-containing portions, and deformability of the flexible tube 11 is good. Also, a thickness T2 of each of microparticle-free portions that are inward of the first and second microparticle-containing portions 13a and 13b shown in Fig. 6 is preferably 1/5 to 1/3 of the thickness of the flexible tube 11. Also, a thickness T3 of each of microparticle-free portions that are outward of the first and second microparticle-containing portions 13a and 13b shown in Fig. 6 is preferably 1/5 to 1/3 of the thickness of the flexible tube 11.

As shown in Figs. 4 through 6, the first and second microparticle-containing portions 13a and 13b are extended from the distal end of the flexible tube 11 toward the proximal end thereof in the predetermined width and in parallel with the central axis of the flexible tube 11. In the embryo transfer tool of this embodiment, the first and second microparticle-containing portions 13a and 13b are extended toward the proximal end of the flexible tube in a substantially constant width. As shown in Fig. 6, a width of each of the first and second microparticle-containing portions 13a and 13b is set to 5/100 to 20/100 of the outer circumferential length of the flexible tube 11. By setting the width of each of the first and second microparticle-containing portions 13a and 13b to the above-described range, the first and second colorless and transparent parts 15a and 15b are allowed to have a sufficiently large width. It is preferable to set the width of each of the first and second microparticle-containing portions 13a and 13b to 7/100 to 15/100 of the outer circumferential length of the flexible tube 11. An angle R1 of a part where the first microparticle-containing portion 13a is formed and that of a part where the second microparticle-containing portion 13b is formed are set to favorably 15 to 70 degrees and more favorably 30 to 50 degrees. Although it is preferable to set the width of the first microparticle-containing portion 13a and that of the second microparticle-containing portion 13b substantially equally to each other, it is possible to differentiate the widths thereof from each other.

The embryo transfer tool 1 has the first colorless and transparent part 15a positioned between the first and second microparticle-containing portions 13a and 13b and extended in the predetermined length from the distal end of the flexible tube 11 toward the proximal end thereof and the second colorless and transparent part 15b provided opposite to the first colorless and transparent part 15a through the intermediary of the central axis of the flexible tube. The first and second colorless and transparent parts 15a and 15b are provided opposite to each other and have sufficiently high optical transparency and microscopic visibility inside the lumen 16. Thereby it is possible to check the presence of living cells held inside the lumen by a microscope.

The width of each of the colorless and transparent parts 15a and 15b is set to not less than 30/100 of the outer circumferential length of the flexible tube 11. It is preferable to set the width of each of the colorless and transparent parts 15a and 15b to not less than 35/100 of the outer circumferential length of the flexible tube 11. It is favorable to set an angle R2 of a region in which the first colorless and transparent part 15a is formed and that of a region in which the second colorless and transparent part 15b is formed to 110 to 165 degrees and more favorable to set the angle R2 to 130 to 150 degrees. Although it is preferable to set the widths of the first and second colorless and transparent parts 15a and 15b substantially equally to each other, the widths thereof may be differentiated from each other.

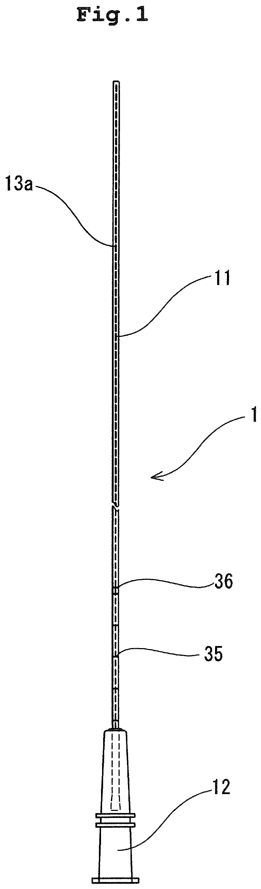

As shown in Fig. 1, the embryo transfer tool 1 of this embodiment has a plurality of markers 35 arranged at regular intervals at the proximal end portion of the flexible tube 11, which is located distally from the hub 12. More specifically, the embryo transfer tool 1 has five distance index markers 35 arranged at equal intervals and an end marker 36 positioned proximately to one distance index marker 35 located most distally and distally therefrom.

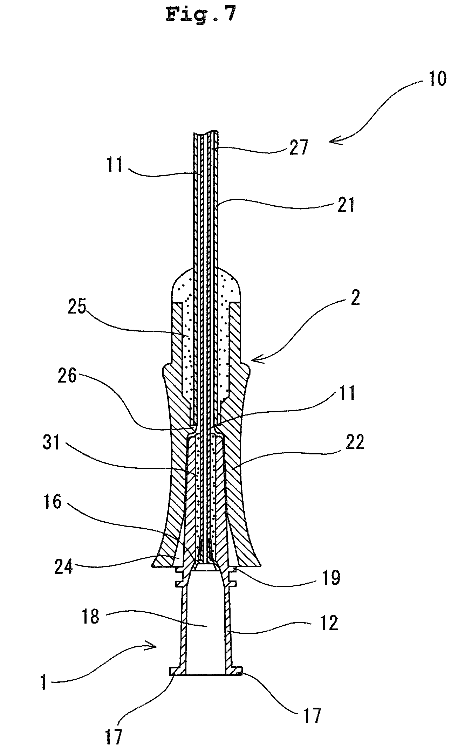

The embryo transfer tool 1 has the hub 12 fixed to the proximal end portion of the flexible tube 11. As shown in Fig. 7, the flexible tube 11 is fixed to the hub 12 with a caulking member 16 inserted into the proximal end of the flexible tube and an adhesive agent 31.

The hub 12 has an open part at its proximal end and a hollow part 18 communicating with the inside of the flexible tube 11. The hollow part 18 of the hub 12 is formed as a luer tapered part which can be liquid-tightly mounted on a nozzle of a medical appliance such as a syringe. The hub 12 has two opposed projected parts 17 projected outward from the proximal end thereof. The hub 12 has two annular ribs 19 provided at its central portion. A part of the hub positioned forward from the rib 19 is formed as a tubular part smaller in its diameter than a part thereof positioned rearward from the rib 19. The small-diameter tubular part of the hub can be inserted into a sheath hub 22 of the sheath 2 to be described later. Hard resin is used as the material for forming the hub.

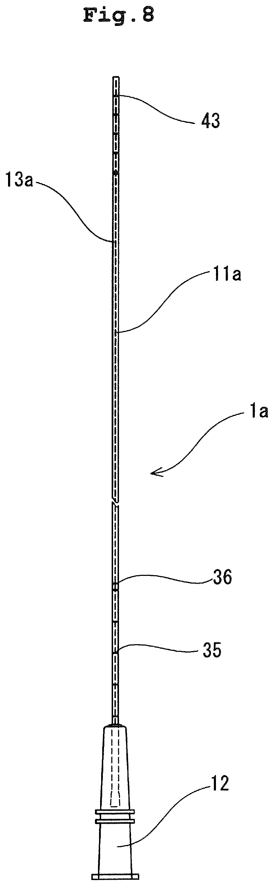

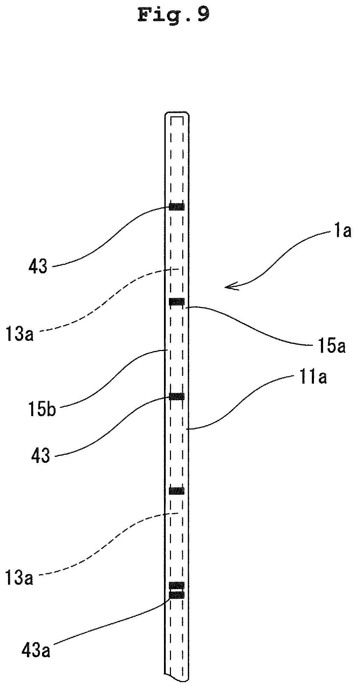

Furthermore, the embryo transfer tool of the present invention may include markers 43 in the distal end portion, as is the case with an embryo transfer tool 1a of an embodiment shown in Fig. 8. More specifically, as shown in Figs. 8 and 9, it is preferable that a flexible tube 11a includes a plurality of markers 43 that are arranged at substantially equal intervals on the outer surface of the distal end portion. The flexible tube shown in Figs. 8 and 9 includes five markers 43 that are arranged at equal intervals and an emphasizing marker 43a that is provided on the proximal end side in the vicinity of the marker 43 that is closest to the proximal end. The number of markers 43 is preferably 3 to 10, and one emphasizing marker 43a is preferably provided for every five markers 43.

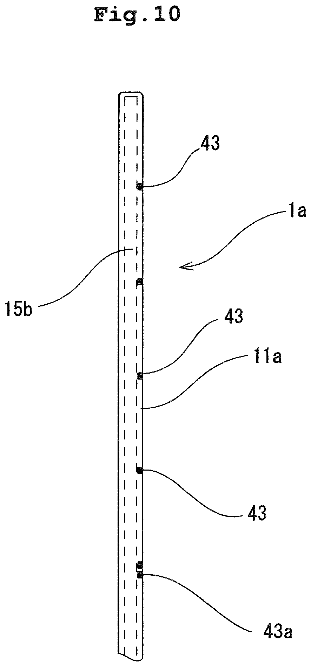

As shown in Fig. 9, each of the markers 43 and 43a is not annular and has the shape of a short band extending in the circumferential direction. Furthermore, the markers are provided so as to be positioned over the first microparticle-containing portion 13a (or the second microparticle-containing portion 13b) of the flexible tube 11a. Accordingly, as shown in Fig. 10, the markers 43 and 43a are not positioned in the first and second colorless and transparent portions 15a and 15b of the flexible tube 11a, and do not impair visibility of the inside of the first and second colorless and transparent portions 15a and 15b.

An embryo transfer device 10 of the present invention is described below.

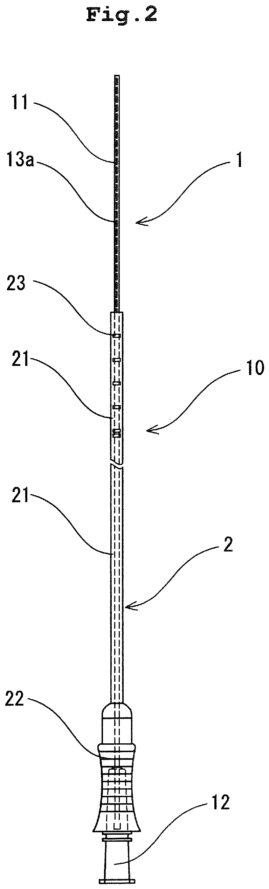

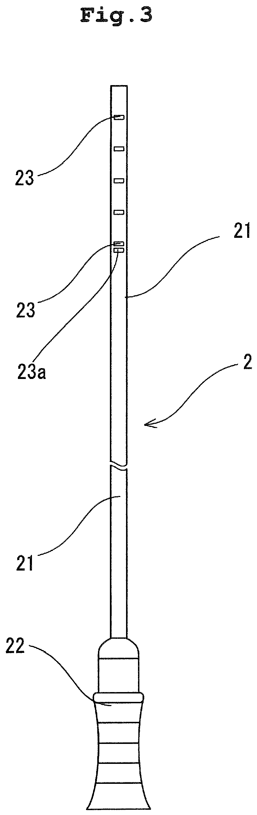

The embryo transfer device 10 is composed of the embryo transfer tool 1 or 1a and the sheath 2. As shown in Figs. 2 and 3, the sheath 2 accommodates the flexible tube 11 or 11a with the distal part of the flexible tube 11 or 11a of the embryo transfer tool 1 projecting from the distal end of the sheath. The sheath has the flexible tube 21 harder than the flexible tube 11 and the sheath hub 22 provided at the proximal end of the flexible tube 21.

The sheath 2 of this embodiment has the flexible tube 21 having a lumen 27 penetrating therethrough from its distal end to its proximal end and the sheath hub 22 fixed to the proximal end portion of the flexible tube 21. The sheath hub 22 may be formed integrally with the flexible tube 21.

As shown in Figs. 2, 3, and 7, the sheath 2 has the flexible tube 21 having the lumen 27 penetrating therethrough from its distal end to its proximal end. The length of the flexible tube 21 is set to 50 to 300mm and preferably 100 to 250mm. The outer diameter of the flexible tube is set to 1 to 5mm and preferably 1.5 to 3.5mm. The inner diameter of the flexible tube is set to 0.8 to 4.8mm and preferably 1.3 to 3.3mm. The length of the part of the flexible tube 11 of the embryo transfer tool 1 projected from the distal end of the sheath 2 is set to favorably 30 to 100mm and more favorably 35 to 70mm.

As materials for forming the flexible tube 21, those harder than the material for forming the flexible tube 11 or 11a and having a certain extent of shape-retaining property and flexibility are used. As the materials for forming the flexible tube 21, it is possible to use polyester, polyolefin (for example, polyethylene, polypropylene, ethylene-propylene copolymer), polyamide (for example, nylon 6, nylon 66), polyester (for example, polyethylene terephthalate), and fluororesin (for example, PTFE, ETFE). As shown in Fig. 3, the sheath 2 of this embodiment has a plurality of insertion depth checking markers 23 formed on the outer surface of the distal part thereof. More specifically, the sheath has five distance index markers 23 arranged at regular intervals and an end marker 23a positioned proximately to one distance index marker 23 located most proximally and proximally therefrom.

As shown in Fig. 7, the sheath hub 22 is fixed to a rear end of the flexible tube 21 with an adhesive agent 25. The sheath hub 22 is a hollow hub having a lumen 24 communicating with the lumen 27 formed inside the flexible tube 21. As shown in Figs. 3 and 7, the sheath hub has a gripping concave part on a side surface thereof and a non-slip rib formed on the surface of the concave part. The sheath hub 22 has an annular rib 26 projected inward inside the lumen 27. The proximal end of the flexible tube 21 is in contact with the annular rib 26. The flexible tube 21 is fixed to the sheath hub 22 with adhesive 25 filled in a space between the sheath hub 22 and the proximal end portion of the flexible tube 21. The diameter of an open portion of the sheath hub 22 increases in a tapered manner. Hard resin is used as a material for forming the sheath hub.

In particular, the first and second microparticle-containing portions are positioned within the inner wall of the flexible tube and are not exposed in the outer surface and the inner surface of the flexible tube, and therefore, both the inner surface and the outer surface of the tube have good smoothness. Furthermore, the first and second microparticle-containing portions are constituted by a synthetic resin and a large number of microparticles that are formed of a material different from the synthetic resin, and the first and second microparticle-containing portions include a large number of boundary surfaces formed between the synthetic resin and the microparticles, and therefore, a uniform and good ultrasound imaging property is achieved. Furthermore, as a result of the first colorless and transparent portion and the second colorless and transparent portion being provided so as to opposed to each other, sufficiently high optical transparency and visibility are achieved. Therefore, it is possible to perform embryo transfer in a favorable manner.

## Claims

1. An embryo transfer tool (1, 1a) comprising:
a flexible tube (11, 11a) that is made of a colorless and transparent first synthetic resin; and
a hub (12) that is provided at a proximal end portion of the flexible tube (11, 11a),
wherein the flexible tube (11, 11a) includes:
a first microparticle-containing portion (13a) that has a predetermined width and extends over a predetermined length from a distal end portion of the flexible tube (11, 11a) toward a proximal end thereof; a second microparticle-containing portion (13b) that has a predetermined width, extends from the distal end portion toward the proximal end, and opposed to the first microparticle-containing portion (13a); a first colorless and transparent portion (15a) that is positioned between the first microparticle-containing portion (13a) and the second microparticle-containing portion (13b), extends over a predetermined length from a distal end of the flexible tube (21) toward the proximal end, and allows the inside of a lumen (16, 24, 27) of the flexible tube (11, 11a) to be visually recognized; and a second colorless and transparent portion (15b) that is provided so as to be opposed to the first colorless and transparent portion (15a),
the first and second microparticle-containing portions (13a, 13b) are positioned within an inner wall of the flexible tube (11, 11a), are not exposed in an outer surface and an inner surface of the flexible tube (11, 11a),
and are constituted by a second synthetic resin and a large number of microparticles (14) that are formed of a material different from the second synthetic resin of which the first and second microparticle-containing portions (13a, 13b) are constituted, the microparticles (14) are dispersed in the second synthetic resin, and the first and second microparticle-containing portions (13a, 13b) include a large number of boundary surfaces that are formed between the second synthetic resin and the microparticles (14), the microparticles (14) have a diameter of 0.5 to 200 µm, and the microparticles (14) are optically transparent hollow glass beads;
wherein
the second synthetic resin forming the first and second microparticle-containing portions (13a, 13b) has higher flexibility than the first synthetic resin forming the rest of the flexible tube (11, 11a),
a material forming the first and second microparticle-containing portions (13a, 13b), which is constituted by the microparticles (14) and the second synthetic resin, has a flexibility that is substantially equivalent to flexibility of the first synthetic resin forming the rest of the flexible tube (11, 11a).

2. The embryo transfer tool (1, 1a) according to claim 1,
wherein the embryo transfer tool (1, 1a) includes, at a distal end portion thereof, an annular transparent distal end portion (11a) that does not include the first and second microparticle-containing portions (13a, 13b).

3. The embryo transfer tool (1, 1a) according to any one of claims 1 to 2,
wherein a thickness of each of the first and second microparticle-containing portions (13a, 13b) is 1/5 to 113 of a thickness of the flexible tube.

4. The embryo transfer tool (1, 1a) according to any one of claims 1 to 3, wherein a width of each of the first and second microparticle-containing portions (13a, 13b) is 5/100 to 20/100 of an outer circumferential length of the flexible tube (11, 11a), and a width of each of the first and second colorless and transparent portions (15a 15b) is at least 30/100 of the outer circumferential length of the flexible tube (11, 11a).

5. The embryo transfer tool (1, 1a) according to any one of claims 1 to 4,
wherein the flexible tube (11, 11a) includes a plurality of markers (43) that are arranged at substantially equal intervals on an outer surface of the distal end portion, the markers (43, 43a) each having the shape of a short band extending in a circumferential direction, being positioned over the first microparticle-containing portion (13a) or the second microparticle-containing portion (13b), and not being positioned over the first and second colorless and transparent portions (15a, 15b).

6. An embryo transfer device (10) comprising:
the embryo transfer tool (1, 1a) according to any one of claims 1 to 5; and
a sheath (2) that includes a flexible tube (21) that is harder than the flexible tube (11, 11a) of the embryo transfer tool (1) and a sheath hub (22) that is provided at a proximal end of the flexible tube (21), the sheath (2) accommodating the flexible tube (11, 11a) of the embryo transfer tool (1, 1a) with the distal end portion of the flexible tube (11, 11a) projecting from the sheath (2).

7. The embryo transfer device (10) according to claim 6,
wherein the flexible tube (11, 11a) of the embryo transfer tool (1, 1a) includes the first and second microparticle-containing portions (13a, 13b) over almost the entire length of a portion of the flexible tube (11, 11a) that projects from a distal end of the sheath (2).

## Patentansprüche

1. Embryotransfer-Instrument (1, 1a), umfassend:
einen flexiblen Schlauch (11, 11a), der aus einem farblosen und transparenten ersten Kunstharz hergestellt ist; und
eine Nabe (12), die an einem proximalen Endabschnitt des flexiblen Schlauchs (11, 11a) vorgesehen ist,
wobei der flexible Schlauch (11, 11a) umfasst:
einen ersten Mikropartikel enthaltenden Abschnitt (13a), der eine vorbestimmte Breite aufweist und sich über eine vorbestimmte Länge von einem distalen Endabschnitt des flexiblen Schlauchs (11, 11a) in Richtung zu einem proximalen Ende davon erstreckt; einen zweiten Mikropartikel enthaltenden Abschnitt (13b), der eine vorbestimmte Breite aufweist, sich von dem distalen Endabschnitt in Richtung zu dem proximalen Ende erstreckt und dem ersten Mikropartikel enthaltenden Abschnitt (13a) gegenüberliegt; einen ersten farblosen und transparenten Abschnitt (15a), der zwischen dem ersten Mikropartikel enthaltenden Abschnitt (13a) und dem zweiten Mikropartikel enthaltenden Abschnitt (13b) angeordnet ist, sich über eine vorbestimmte Länge von einem distalen Ende des flexiblen Schlauchs (21) in Richtung des proximalen Endes erstreckt und es ermöglicht, dass das Innere eines Lumens (16, 24, 27) des flexiblen Schlauchs (11, 11a) visuell zu erkennen ist; und einen zweiten farblosen und transparenten Abschnitt (15b), der so vorgesehen ist, dass er dem ersten farblosen und transparenten Abschnitt (15a) gegenüberliegt,
die ersten und zweiten Mikropartikel enthaltenden Abschnitte (13a, 13b) innerhalb einer Innenwand des flexiblen Schlauchs (11, 11a) angeordnet sind,
an einer Außenfläche und einer Innenfläche des flexiblen Schlauchs (11, 11a) nicht freiliegen und aus einem zweiten Kunstharz und einer großen Anzahl von Mikropartikeln (14) gebildet sind, die aus einem Material hergestellt sind, das sich von dem zweiten Kunstharz unterscheidet, aus dem die ersten und zweiten Mikropartikel enthaltenden Abschnitte (13a, 13b) gebildet sind, die Mikropartikel (14) in dem zweiten synthetischen Kunstharz dispergiert sind und die ersten und zweiten Mikropartikel enthaltenden Abschnitte (13a, 13b) eine große Anzahl von Grenzflächen aufweisen, die zwischen dem zweiten Kunstharz und den Mikropartikeln (14) ausgebildet sind, die Mikropartikel (14) einen Durchmesser von 0,5 bis 200 µm aufweisen, und die Mikropartikel (14) optisch transparente hohle Glaskugeln sind;
wobei
das zweite Kunstharz, das die ersten und zweiten Mikropartikel enthaltenden Abschnitte (13a, 13b) bildet, eine höhere Flexibilität aufweist als das erste Kunstharz, das den Rest des flexiblen Schlauchs (11, 11a) bildet,
ein Material, das die ersten und zweiten Mikropartikel enthaltenden Abschnitte (13a, 13b) bildet, das aus den Mikropartikeln (14) und dem zweiten Kunstharz besteht, eine Flexibilität aufweist, die im Wesentlichen der Flexibilität des ersten Kunstharzes entspricht, das den Rest des flexiblen Schlauchs (11, 11a) bildet.

2. Embryotransfer-Instrument (1, 1a) nach Anspruch 1, wobei das Embryotransfer-Instrument (1, 1a), an einem distalen Endabschnitt davon, einen ringförmigen, transparenten distalen Endabschnitt (11a) aufweist, der die ersten und zweiten Mikropartikel enthaltenden Abschnitte (13a, 13b) nicht einschließt.

3. Embryotransfer-Instrument (1, 1a) nach einem der Ansprüche 1 bis 2, wobei eine Dicke von jedem der ersten und zweiten Mikropartikel enthaltenden Abschnitte (13a, 13b) jeweils 1/5 bis 1/3 der Dicke des flexiblen Schlauchs beträgt.

4. Embryotransfer-Instrument (1, 1a) nach einem der Ansprüche 1 bis 3, wobei eine Breite von jedem der ersten und zweiten Mikropartikel enthaltenden Abschnitte (13a, 13b) 5/100 bis 20/100 einer äußeren Umfangslänge des flexiblen Schlauchs (11, 11a) beträgt, und eine Breite von jedem der ersten und zweiten farblosen und transparenten Abschnitte (15a, 15b) mindestens 30/100 der äußeren Umfangslänge des flexiblen Schlauchs (11, 11a) beträgt.

5. Embryotransfer-Instrument (1, 1a) nach einem der Ansprüche 1 bis 4,
wobei der flexible Schlauch (11, 11a) eine Vielzahl von Markierungen (43) aufweist, die in im Wesentlichen gleichen Abständen auf einer Außenfläche des distalen Endabschnitts angeordnet sind, wobei die Markierungen (43, 43a) jeweils die Form eines kurzen, sich in Umfangsrichtung erstreckenden Bandes aufweisen, das über dem ersten Mikropartikel enthaltenden Abschnitt (13a) oder dem zweiten Mikropartikel enthaltenden Abschnitt (13b) angeordnet ist und nicht über dem ersten und zweiten farblosen und transparenten Abschnitt (15a, 15b) angeordnet ist.

6. Vorrichtung (10) für den Embryotransfer, umfassend:
das Embryotransfer-Instrument (1, 1a) nach einem der Ansprüche 1 bis 5; und
eine Hülle (2), die einen flexiblen Schlauch (21), der härter ist als der flexible Schlauch (11, 11a) des Embryotransfer-Instrumentes (1), und eine Hüllennabe (22) umfasst, die an einem proximalen Ende des flexiblen Schlauchs (21) vorgesehen ist,
die Hülle (2) den flexiblen Schlauch (11, 11a) des Embryotransfer-Instrumentes (1, 1a) aufnimmt, wobei der distale Endabschnitt des flexiblen Schlauchs (11, 11a) aus der Hülle (2) herausragt.

7. Vorrichtung (10) für den Embryotransfer nach Anspruch 6,
wobei der flexible Schlauch (11, 11a) des Embryotransfer-Instrumentes (1, 1a) die ersten und zweiten Mikropartikel enthaltenden Abschnitte (13a, 13b) über fast die gesamte Länge eines Abschnitts des flexiblen Schlauchs (11, 11a) aufweist, der aus einem distalen Ende der Hülle (2) herausragt.

## Revendications

1. Outil de transfert d'embryon (1, 1a) comprenant :
un tube flexible (11, 11a) qui est composé d'une première résine synthétique incolore et transparente ; et
un moyeu (12) qui est prévu au niveau d'une partie d'extrémité proximale du tube flexible (11, 11a),
dans lequel le tube flexible (11, 11a) comporte :
une première partie contenant des microparticules (13a) qui présente une largeur prédéterminée et s'étend sur une longueur prédéterminée à partir d'une partie d'extrémité distale du tube flexible (11, 11a) en direction d'une extrémité proximale de celui-ci ; une deuxième partie contenant des microparticules (13b) qui présente une largeur prédéterminée, s'étend à partir de la partie d'extrémité distale en direction de l'extrémité proximale, et opposée à la première partie contenant des microparticules (13a) ; une première partie incolore et transparente (15a) qui est positionnée entre la première partie contenant des microparticules (13a) et la deuxième partie contenant des microparticules (13b), s'étend sur une longueur prédéterminée à partir d'une extrémité distale du tube flexible (21) en direction de l'extrémité proximale, et permet à l'intérieur d'une lumière (16, 24, 27) du tube flexible (11, 11a) d'être visuellement reconnu ; et une deuxième partie incolore et transparente (15b) qui est prévue de manière à être opposée à la première partie incolore et transparente (15a),
les première et deuxième parties contenant des microparticules (13a, 13b) sont positionnées au sein d'une paroi intérieure du tube flexible (11, 11a), ne sont pas exposées dans une surface extérieure et une surface intérieure du tube flexible (11, 11a), et sont constituées par une deuxième résine synthétique et un grand nombre de microparticules (14) qui sont formées d'un matériau différent de la deuxième résine synthétique dont les première et deuxième parties contenant des microparticules (13a, 13b) sont constituées, les microparticules (14) sont dispersées dans la deuxième résine synthétique, et les première et deuxième parties contenant des microparticules (13a, 13b) comportent un grand nombre de surfaces limites qui sont formées entre la deuxième résine synthétique et les microparticules (14), les microparticules (14) présentent un diamètre de 0,5 à 200 um, et les microparticules (14) sont des billes de verre creuses optiquement transparentes ;
dans lequel
la deuxième résine synthétique formant les première et deuxième parties contenant des microparticules (13a, 13b) présente une flexibilité plus élevée que la première résine synthétique formant le reste du tube flexible (11, 11a), un matériau formant les première et deuxième parties contenant des microparticules (13a, 13b), qui est constitué par les microparticules (14) et la deuxième résine synthétique, présente une flexibilité qui est sensiblement équivalente à la flexibilité de la première résine synthétique formant le reste du tube flexible (11, 11a).

2. Outil de transfert d'embryon (1, 1a) selon la revendication 1,
dans lequel l'outil de transfert d'embryon (1, 1a) comporte, au niveau d'une partie d'extrémité distale de celui-ci, une partie d'extrémité distale transparente annulaire (11a) qui ne comporte pas les première et deuxième parties contenant des microparticules (13a, 13b).

3. Outil de transfert d'embryon (1, 1a) selon l'une quelconque des revendications 1 à 2,
dans lequel une épaisseur de chacune des première et deuxième parties contenant des microparticules (13a, 13b) est 1/5 à 1/3 d'une épaisseur du tube flexible.

4. Outil de transfert d'embryon (1, 1a) selon l'une quelconque des revendications 1 à 3, dans lequel une largeur de chacune des première et deuxième parties contenant des microparticules (13a, 13b) est 5/100 à 20/100 d'une longueur circonférentielle extérieure du tube flexible (11, 11a), et une largeur de chacune des première et deuxième parties incolores et transparentes (15a, 15b) est au moins 30/100 de la longueur circonférentielle extérieure du tube flexible (11, 11a).

5. Outil de transfert d'embryon (1, 1a) selon l'une quelconque des revendications 1 à 4,
dans lequel le tube flexible (11, 11a) comporte une pluralité de marqueurs (43) qui sont agencés à intervalles sensiblement égaux sur une surface extérieure de la partie d'extrémité distale, les marqueurs (43, 43a) présentant chacun la forme d'une bande courte s'étendant dans une direction circonférentielle, positionnée au-dessus de la première partie contenant des microparticules (13a) ou la deuxième partie contenant des microparticules (13b), et non positionnée au-dessus des première et deuxième parties incolores et transparentes (15a, 15b).

6. Dispositif de transfert d'embryon (10) comprenant :
l'outil de transfert d'embryon (1, 1a) selon l'une quelconque des revendications 1 à 5 ; et
une gaine (2) qui comporte un tube flexible (21) qui est plus dur que le tube flexible (11, 11a) de l'outil de transfert d'embryon (1) et un moyeu de gaine (22) qui est prévu au niveau d'une extrémité proximale du tube flexible (21), la gaine (2) recevant le tube flexible (11, 11a) de l'outil de transfert d'embryon (1, 1a) avec la partie d'extrémité distale du tube flexible (11, 11a) se projetant à partir de la gaine (2).

7. Dispositif de transfert d'embryon (10) selon la revendication 6,
dans lequel le tube flexible (11, 11a) de l'outil de transfert d'embryon (1, 1a) comporte les première et deuxième parties contenant des microparticules (13a, 13b) sur presque toute la longueur d'une partie du tube flexible (11, 11a) qui se projette à partir d'une extrémité distale de la gaine (2).
